# EUROPÄISCHE PATENTSCHRIFT

(11) **EP 1 704 895 B1**
(45) Veröffentlichungstag und Bekanntmachung des Hinweises auf die Patenterteilung: **05.10.2011**
(21) Anmeldenummer: 06005850.0
(22) Anmeldetag: 22.03.2006
(51) Int. Cl.: A61N 2/02, A61N 1/40

(54) **Befeldungsvorrichtung für die Befeldung von Körperteilen von Lebewesen zu Heilzwecken**
Field emitting device for the irradiation of living body parts for therapeutical purposes
Dispositif émettant un champ pour l'irradiation de parties du corps vivant à des fins thérapeutiques

(30) Priorität: 23.03.2005 DE 102005014023
(43) Veröffentlichungstag der Anmeldung: 27.09.2006
(73) Patentinhaber: Mega-Wave-GmbH, 86825 Bad Wörishofen (DE)
(72) Erfinder: Feucht, Peter, 12247 Berlin-Lankwitz (DE)
(74) Vertreter: Liebl, Thomas

(56) Entgegenhaltungen:
- EP-A- 0 136 530
- US-B1- 6 334 069
- US-B1- 6 458 121

## Beschreibung

Die Erfindung betrifft eine Befeldungsvorrichtung für die Befeldung von Körperteilen von Lebewesen zu Heilzwecken definiert durch Anspruch 1.

Eine Befeldungsvorrichtung nach dem Oberbegriff des Anspruchs 1 ist aus US 6334 069 B1 bekannt.

Eine bekannte gattungsgemäße Befeldungsvorrichtung (EP 0 136 530 B1) besteht aus einem Befeldungsgerät mit einem Hochfrequenzsender und mit einem Sollwertsteller zur Vorgabe einer Befeldungs-Feldstärke. Weiter besteht die Befeldungsvorrichtung aus wenigstens einer Befeldungskapsel, die zumindest eine mit dem Hochfrequenzsender verbundene Magnetspule enthält. Die Befeldungskapsel ist über Kabel am Befeldungsgerät angeschlossen und kann zu Therapiezwecken an einem Körperteil eines Lebewesens angelegt werden.

Konkret wird mit der bekannten Befeldungsvorrichtung zur Erzielung therapeutischer Effekte eine Hochfrequenzenergie mit einer Trägerfrequenz im Bereich um 150 MHz abgestrahlt, um mit geringen, athermischen magnetischen Feldstärken biologische Zellen zu beeinflussen. Trotz der geringen Feldstärken im Bereich von ca. pA/m können im Bereich der Befeldungskapsel oder gegebenenfalls mehrerer Befeldungskapseln Grenzwerte für die zulässige Funkstörfeldstärke überschritten werden. Die gemessenen Hochfrequenz-Störpegel (HF-Störpegel) können dabei etwa 20 bis 40 dB über den zulässigen Grenzwerten liegen. Da im Bereich von 150 MHz Funkfrequenzen für den Mobilfunk, den GPS-Betrieb, etc. liegen, ist die Einhaltung der vorgegebenen Grenzwerte zwingend geboten.

Eine Abschirmung der Behandlungsräume zur Einhaltung der vorstehenden Grenzwerte in der weiteren Umgebung stellt einen nicht vertretbaren Aufwand dar, der einen vielfachen Wert des Befeldungsgeräts ausmachen würde. Die Anwendung in nicht speziell abgeschirmten Arztpraxen oder beim Patienten zuhause wäre nicht zulässig. Eine starke Absenkung der Befeldungsfeldstärke zur sicheren Einhaltung noch zulässiger HF-Störpegel würde den erzielbaren medizinischen Nutzen des Befeldungsverfahrens erheblich reduzieren.

Aufgabe der Erfindung ist es, eine gattungsgemäße Befeldungsvorrichtung so weiterzubilden, dass von der Befeldungskapsel in die Umgebung abgestrahlte Hochfrequenz-Störpegel bei allen Betriebsmöglichkeiten sicher unter einem vorgebbaren unkritischen Wert gehalten werden. Diese HF-Störpegel sollen so gering gehalten werden, dass beispielsweise die Auflagen der deutschen Behörde "Bundes-Netzagentur" bezüglich Störfeldgrößen erfüllt werden können und die sog. "technische Unbedenklichkeit" für die allgemeine Anwendung erhalten werden kann. Inbesondere soll ein unzulässig hoher HF-Störpegel auch dann vermieden werden, wenn beim Einshalten des Befeldungsgeräts die BefeldungsKapsel nicht am Körper anliegt. Diese Aufgabe wird mit den Merkmalen des Anspruchs 1 gelöst.

Es ist eine Indikatoreinheit vorgesehen, welche die vom Hochfrequenzsender an die Befeldungskapsel abgegebene Sendeleistung mit der Größe der dadurch in der Befeldungskapsel erzeugten zugeordneten Befeldungs-Feldstärke direkt oder indirekt vergleicht und ins Verhältnis setzt. Dieses Verhältnis unterscheidet sich in der Funktionsposition und in der Leerlaufposition der Befeldungskapsel. Dieser Unterschied wird messtechnisch erfasst, wodurch sich eine eindeutige Zuordnung für eine Befeldungskapsel in ihrer Leerlaufsposition oder Funktionsposition ergibt. Bei ermittelter Leerlaufposition gibt die Indikatoreinheit ein Schaltsignal ab, mit dem auf einen Sicherheitsbetrieb geschaltet wird, in dem die Befeldungsvorrichtung in einer ersten Alternative bis zu einer Wiedereinschaltung abgeschaltet wird, so dass überhaupt kein Störpegel vorliegt. In einer zweiten Alternative werden der Senderpegel und damit die Feldstärke über eine Feldstärke-Begrenzungseinrichtung soweit auf einen festgelegten Wert heruntergefahren, dass im Leerlauffall kein unzulässig hoher, von der Befeldungskapsel in die Umgebung abgestrahlter Hochfrequenz-Störpegel (HF-Störpegel) auftritt.

Eine Analyse des Problems unzulässig hoher kritischer HF-Störpegel an der Befeldungskapsel zeigt, dass diese insbesondere dann auftreten, wenn mit der/den Magnetspulen, die als magnetische Antennen wirken, in den freien Raum, in einem sog. Leerlaufbetrieb abgestrahlt wird, wobei die Befeldungskapsel nicht auf einem Körperteil eines Lebewesens, insbesondere eines Patienten aufliegt. Durch eine solche Anlage erfolgt eine Kopplung mit einer Dämpfung durch das angrenzende Körpergewebe, wodurch die vom HF-Sender an die Magnetspule gelieferte Leistung in wesentlich geringerem Umfang als HF-Störpegel in die Umgebung abgestrahlt wird, als vergleichsweise im vorstehend genannten Leerlaufbetrieb.

Bei der Befeldungsvorrichtung nach dem aus US 6334069 B1 bekannten Stand der Technik wird mit dem Sollwertsteller der Pegel des Hochfrequenzsenders eingestellt. Durch eine bestimmten Pegeleinstellung wird eine bestimmte therapeutisch vorgegebene Befeldungs-Feldstärke an der Befeldungskapsel erzeugt, jedoch nur für den Fall, dass die Befeldungskapsel gewebegedämpft am Patienten anliegt. Bei einem bestimmungsgemäßen Betrieb mit ordnungsgemäß am Patienten anliegender Befeldungskapsel führt dieser Betrieb zu keinen unzulässig hohen HF-Störpegeln. Dagegen würde im Leerlaufbetrieb ohne Anlage am Patienten bei gleicher Pegeleinstellung für den Hochfrequenzsender die Feldstärke an der Magnetspule/Magnetspulen mit unzulässig hohen, in die Umgebung abgestrahlten HF-Störpegeln stark an steigen. Die von der Magnetspule erzeugte Magnetspulenfeldstärke wird jedach bei einem messtechnisch erkannten, kritischen Leerlaufbetrieb durch einen Sicherheitsbetrieb begrenzt,

Die dazu verwendete Indikatoreinrichtung wirkt dabei wie ein Schalter oder Sensor, der der Befeldungskapsel zugeordnet ist und der deren ordnungsgemäße Anlage an einem Körpergewebe oder den Leerlauffall erkennt und ein entsprechendes Signal zur Verfügung stellt. Die Funktion eines solchen Schalters oder Sensors ist einfach, kostengünstig und funktionssicher durch einen direkten oder indirekten Vergleich der aktuellen Senderpegel mit den dadurch an der Befeldungskapsel bewirkten Feldstärken nachgebildet. Falls von der Indikatoreinrichtung Zustände erfasst werden, die unzulässig hohe HF-Störpegel bedingen, kann am Befeldungsgerät eine optische und/oder akustische Warnung aktiviert werden und/oder das Befeldungsgerät wird abgeschaltet oder die Leistung des HF-Senders wird abgeschaltet oder auf unkritische Werte reduziert. Erfindungsgemäß dient der Sicherheitsbetrieb dazu, dass keine oder nur geringe HF-Störpegel erzeugt werden, wodurch unzulässig hohe, von der Befeldungskapsel in die Umgebung abgestrahlte HF-Störpegel sicher ausgeschlossen werden.

Erfindungsgemäβ weist die Feldstärke-Begrenzungseinrichtung eine Initialisierungseinheit auf, über die in einem Initialisierungsmodus nach dem Einschalten des Befeidungsgeräts der HF-Sender gesteuert mit einem so geringen vorgegebenen Initialsenderpegel sendet, dass in keinem der beiden möglichen Betriebszustände, in der Funktionsposition oder Leerlaufposition der Befeldungskapsel, ein unzulässig hoher HF-Störpegel beim Einschalten an der Befeldungskapsel überschritten wird, wobei dem Initialsenderpegel bei am Körperteil eines Lebewesens anliegender Befeldungskapsel eine bestimmte niedrig bedämpfte Initial-Feldstärke und bei nicht anliegender Befeldungskapsel eine bestimmte höhere Leerlauf-Initial-Feldstärke zugeordnet sind. Einer Grenzwerteinheit ist der Feldstärke-Istwert zugeführt und es ist ein Grenzwert zwischen der bedämpften Initial-Feldstärke und der Leerlauf-Initial-Feldstärke eingestellt. Bei einer Überschreitung des Grenzwerts im Initialisierungsmodus erfolgt eine optische und/oder akustische Wamanzeige, die einen Benutzer auf die fehlende Gewebeanlage der Befeldungskapsel hinweist. Bei einer Unterschreitung des Grenzwerts im Initialisierungsmodus entsprechend einer an einem Körpergewebe anliegenden Befeldungskapsel wird der Initialisierungsmodus beendet und es erfolgt eine Freigabe der Reglereinheit.

Hervorzuheben ist dabei, dass durch den vorstehenden Initialisierungsmodus ein unzulässig hoher HF-Störpegel im Leerlaufbetrieb von vorneherein vermieden wird - ohne Initialisierungsmodus kann im Leerlaufbetrieb nach dem Einschalten des Befeldungsgeräts vorerst ein unzulässig hoher HF-Störpegel erzeugt werden, der dann erst wieder reduziert wird.

Vorteilhaft wird nach Anspruch 2 eine Zeitschaltung verwendet, mit der eine Befeldungszeit vorgebbar ist und die gegebenenfalls den Initialisierungsmodus aktiviert.

Mit den Merkmalen des Anspruchs 3 wird eine Alternative einer konkreten, funktionssicheren Indikatoreinheit angegeben. Dazu ist ein der Befeldungskapsel zugeordneter Feldstärkesensor zur Ermittlung des Feldstärke-Istwerts der Magnetspule vorgesehen, welcher an die Indikatoreinheit und gegebenenfalls als Istwertgeber an eine Regeleinheit angeschlossen ist. Zudem ist ein Pegelsensor zur Ermittlung des aktuellen Senderpegel-Istwerts vorgesehen, der an die Indikatoreinheit für einen Vergleich mit dem zugeordneten Feldstärke-Istwert angeschlossen ist, woraus die Funktionsposition oder Leerlaufposition der Befeldungskapsel ermittelt wird.

In einer Weiterbildung nach Anspruch 4 ist der Feldstärkesensor in der Befeldungskapsel angeordnet, wobei damit der elektrische Strom und/oder die elektrische Spannung der Magnetspule ermittelt werden und die Werte linear oder logarithmisch gleichgerichtet auf einem Signalweg zur Kontrolleinheit bzw. der Indikatoreinheit geleitet werden.

Mit den Merkmalen des Anspruchs 5 wird eine weitere Alternative einer konkreten, funktionssicheren Indikatoreinheit beansprucht. In Verbindung mit der Indikatoreinheit sind ein Feldstärkesensor und ein Regelsensor zu einer mittelbaren und indirekten Ermittlung der Feldstärke im Vergleich zum Senderpegel durch einen am HF-Ausgang des Befeldungsgeräts angeordneten Richtkoppler als Stehwellenmessgerät zur Erfassung eines sog. Stehwellenverhältnisses gebildet, welches in der Funktionsposition und der Leerlaufposition der Befeldungskapsel unterschiedlich ist. Anhand des ermittelten Stehwellenverhältnisses wird festgestellt, ob die Befeldungskapsel in der ordnungsgemäßen Funktionsstellung die HF-Senderleistung gewebegedämpft oder im Leerlauffall ohne Körpergewebeanlage nicht gewebegedämpft abnimmt. Nach Feststellung eines Leerlauffalls erfolgt eine Ansteuerung eines Abschwächers zur Reduzierung des Senderpegels oder die Befeldung wird selbsttätig abgeschaltet.

Die Indikatoreinheit wird vorzugsweise in Verbindung mit einer Regeleinrichtung betrieben. Dazu enthält in einer konkreten Ausführungsform nach Anspruch 6 die Kontrolleinheit eine Reglereinheit, der der Feldstärke-Istwert und ein mit dem Sollwertsteller vorgebbarer Feldstärke-Sollwert zugeführt werden und die nach einem Soll-Istwert-Vergleich über einen vorgebbaren Regelalgorithmus ein Stellsignal abgibt. Grundsätzlich können ein Senderpegel des HF-Senders oder vorzugsweise die an der Magnetspule auftretende Feldstärke geregelt werden.

Es wird vorgeschlagen, die Befeldungsvorrichtung von vorneherein so zu dimensionieren, dass über den Sollwertsteller nur maximal so große Befeldungs-Feldstärken vorgebbar sind, die bei einer an einem Körpergewebe eines Lebewesens anliegenden Befeldungskapseln dort zu keinen unzulässig hohen in die Umgebung abgestrahlten HF-Störpegeln führen.

Der HF-Sender, die Kontrolleinheit mit der Indikatoreinheit und der Regeleinheit und die Sender-Abschwächereinheit sowie gegebenenfalls die Initialisierungseinheit und Bedien- und Anzeigeeinheit können in einem kompakten Befeldungsgerät enthalten sein. An das Befeldungsgerät können nach Anspruch 7 eine oder mehrere Befeldungskapseln über Koaxialkabel für eine bewegliche Handhabung angeschlossen sein. Wenn mehrere Befeldungskapseln an einem Befeldungsgerät angeschlossen sind, ist jeder Befeldungskapsel eine Kontrolleinheit mit Indikatoreinheit zuzuordnen.

Gute therapeutische Wirkungen werden erzielt, wenn nach Anspruch 8 der HF-Oszillator ein HF-Signal mit einer vorgebbaren Frequenz im Bereich von 100 MHz bis 200 MHz, bevorzugt von ca. 150 MHz erzeugt. Zudem kann das HF-Signal mit einer vorgebbaren Niederfrequenz moduliert und/oder mit einer vorgebbaren Taktfrequenz getaktet werden.

Anhand einer Zeichnung wird die Erfindung näher erläutert.

Es zeigen:
- Fig. 1: ein Blockschaltbild einer ersten Ausführungsform einer Befel- dungsvorrichtung, und
- Fig. 2: ein Blockschaltbild einer zweiten Ausführungsform einer Befel- dungsvorrichtung.

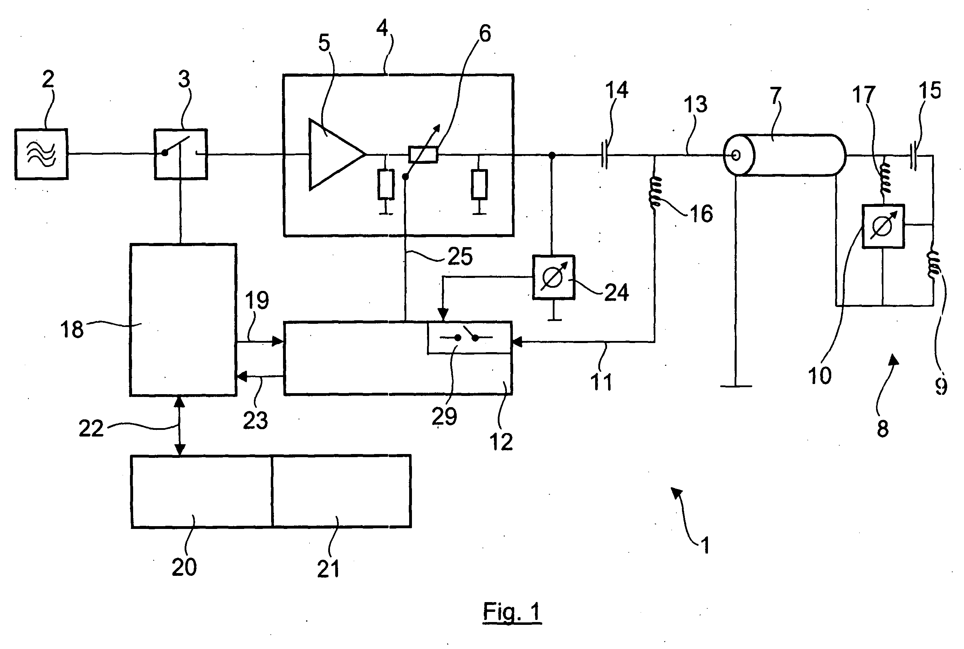

Das Blockschaltbild nach Fig. 1 betrifft eine erste Ausführungsform einer Befeldungsvorrichtung 1, mit einem Hochfrequenz-Oszillator 2, der eine Trägerfrequenz von 150 MHz erzeugt. Dem Hochfrequenz-Oszillator 2 ist ein Schalter 3 zum Ein- und Ausschalten des Befeldungsvorgangs nachgeschaltet, wobei der Schalter 3 mit einer (nicht dargestellten) Zeitschaltung gekoppelt sein kann und schematisch für eine Modulation mit niedrigen Frequenzen und/oder einer Taktung zu Wellenpaketen für die Erzeugung der biologischen Wirkung stehen soll. An den Schalter 3 schließt sich eine Sender-Abschwächer-Einheit 4 an, in der einem HF-Sender 5 ein schematisch durch einen steuerbaren veränderlichen Widerstand dargestellter Abschwächer 6 nachgeordnet ist. Konkret kann diese Einheit durch einen sog. "Voltage controlled gain amplifier" (VGA) realisiert werden.

An die Sender-Abschwächereinheit 4 ist über ein Koaxialkabel 7 eine Befeldungskapsel 8 angeschlossen. Diese ist schematisch als Schaltbild gezeigt, konkret jedoch als Gegenstand, der auf ein Körperteil auflegbar ist, ausgeführt. In der Befeldungskapsel ist eine Magnetspule 9 als Befeldungsspule in der Art einer magnetischen Antenne enthalten. Zudem enthält die Befeldungskapsel 8 einen Feldstärkesensor 10, der den elektrischen Strom und/oder die elektrische Spannung in der Magnetspule 9 ermittelt. Diese Werte werden linear oder logarithmisch gleichgerichtet auf einem Signalweg 11 einer Kontrolleinheit 12 zugeführt. Die Kontrolleinheit 12 enthält eine Indikatoreinheit 29 mit einem Vergleicher sowie eine übliche Regeleinheit.

Das (nur in einem Teilabschnitt dargestellte) Koaxialkabel 7 wird dreifach genutzt, erstens indem der Hochfrequenzweg 13 von der Sender-Abschwächereinheit 4 zur Magnetspule 9 über die Kondensatoren 14, 15 verläuft, zweitens die Betriebsspannungsversorgung für die Sensorschaltung aufgebracht und drittens zudem der Signalweg 11 vom Feldstärkesensor 10 zur Kontrolleinheit 12 für den ermittelten Feldstärke-Istwert über Drosseln 16, 17 entkoppelt werden.

Der Kontrolleinheit 12 wird über den Signalweg 11 ein Feldstärke-Istwert und über einen Signalweg 19 aus einem Mikrocontroller 18 ein vorgebbarer Fe!d-stärke-Sollwert zugeführt wird. Eine Bedieneinheit 20 und Anzeigeeinheit 21 sind über eine Signalleitung 22 für einen Datenaustausch mit dem Mikrocontroller 18 verbunden, dem zudem über eine Leitung 23 Daten aus der Kontrolleinheit 12, beispielsweise in einem Initialisierungsmodus, zugeführt werden. Weiter wird der Senderpegel nach der Sender-Abschwächereinheit 4 mit einem Pegelsensor 24 erfasst und über eine Signalleitung 30 der Kontrolleinheit 12 zugeführt. Ein Ausgang der Kontrolleinheit 12 ist mit einer Stellleitung 25 als Steuerleitung mit dem Abschwächer 6 verbunden.

Die Anordnung hat folgende Funktion: Über die Sender-Abschwächereinheit 4 wird ein niederfrequent moduliertes Hochfrequenzsignal mit 150 MHz der Magnetspule 9 als Befeldungsspule zugeführt, wobei der Feldstärke-Sollwert nach medizinischen Erfordernissen über den Signalweg 19 der Kontrolleinheit 12 zugeführt wird. Die in der Magnetspule 9 erzeugte Feldstärke wird vom Feldstärkesensor 10 ermittelt und als Feldstärke-Istwert über den Signalweg 11 an die Kontrolleinheit 12 zuerst in deren Indikatoreinheit 29 zurückgeführt. Zudem wird der Senderpegel vom Pegelsensor 24 nach der Sender-Abschwächereinheit 4 als aktueller Senderpegel-Istwert ermittelt und ebenfalls der Indikatoreinheit 29 zugeführt. Jedem aktuellen Senderpegel-Istwert entspricht innerhalb geringer Grenzen ein bestimmter, zugeordneter aktueller Feldstärke-Istwert, deren jeweilige Größe durch die konkrete Bauart der Befeldungsvorrichtung gegeben ist. Im Fall einer ordnungsgemäß an einem Körperteil in ihrer Funktionsstellung anliegenden Befeldungskapsel 8 können entsprechende aktuelle Senderpegel-Istwerte und zugehörige Feldstärke-Istwerte empirisch ermittelt und beispielsweise in einer Speichermatrix abgelegt werden. Im Betriebsfall wird von der Indikatoreinheit 29 stets mit einer Vergleichereinheit abgeprüft, ob diese Zuordnung vorliegt. Wenn ja, ist dies die indirekte Ermittlung, dass die Befeldungsspule ordnungsgemäß in ihrer Funktionsstellung an einem Körperteil anliegt. Die Indikatoreinheit 29 gibt die Regeleinheit in der Kontrolleinheit 12 zur Regel der Feldstärke frei. Dort wird ein Soll-Istwert-Vergleich durchgeführt. Bei einer Abweichung wird gegebenenfalls unter Einbeziehung eines Regelalgortihmus, der im Mikrocontroller 18 abgelegt sein kann, ein Steuersignal für den Abschwächer 6 erzeugt. Bauartbedingt tritt dabei kein unzulässig hoher HF-Störpegel in der Umgebung auf.

Stellt im Betriebsfall die Indikatoreinheit 29 eine Abweichung von oben genannter Zuordnung fest, insbesondere einen wesentlich höhere Feldstärke-Istwert zu einem bestimmten Senderpegel-Istwert als den zugeordneten abgelegten Feldstärke-Istwert, ist dies die sichere Erkennung eines Leerlauffalls ohne Bedämpfung bei nicht ordnungsgemäß an einem Körperteil anliegender Befeldungskapsel. In diesem Fall wird die übliche Regelung nicht freigegeben bzw. abgeschaltet und statt dessen der Senderpegel-Istwert und damit der Feldstärke-Istwert unter einen auch im Leerlauffall in der Umgebung unkritischen HF-Störpegel über den Abschwächer 6 heruntergefahren. Alternativ kann die Befeldungsvorrichtung auch abgeschaltet werden und kann dann erst wieder mit am Körperteil anliegender Befeldungskapsel gestartet werden.

Die Indikatoreinheit 29 entspricht somit indirekt einem Schalter, der auf das Körpergewebe anspricht und mit dem im erkannten Leerlauffall von der üblichen Feldstärkeregelung auf eine andere unkritische Betriebsweise umgeschaltet wird.

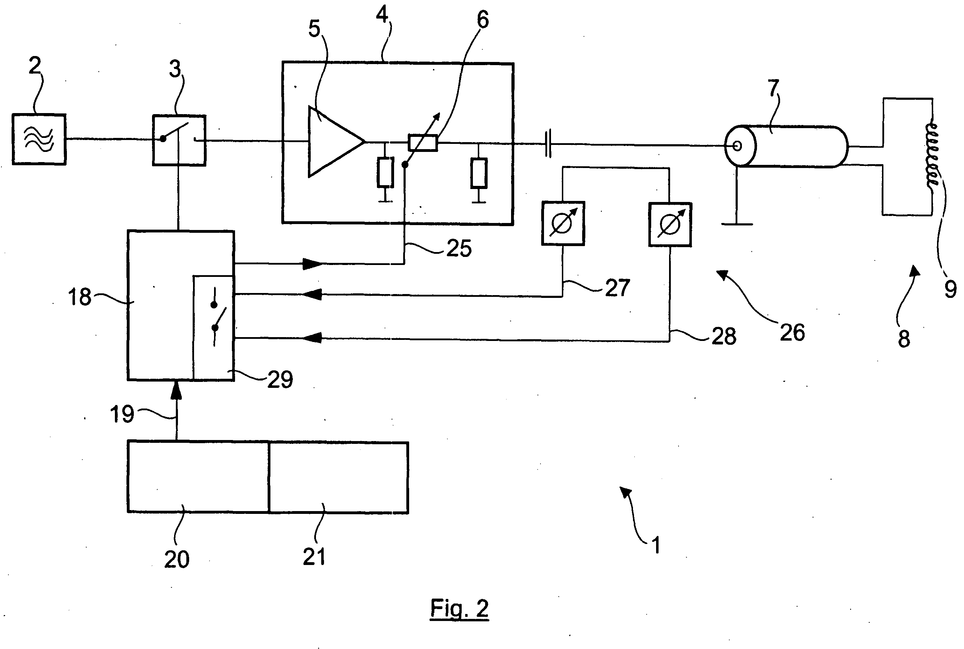

Eine alternative zweite Ausführungsform einer Befeldungsvorrichtung 1 nach Fig. 2 ist ähnlich der Ausführungsform nach Fig. 1 aufgebaut, so dass für gleiche Teile gleiche Bezugszeichen verwendet sind. Auch in dieser zweiten Ausführungsform sind ein Hochfrequenz-Oszillator 2, ein Schalter 3, eine Sender-Abschwächereinheit 4 mit einem HF-Sender 5 und einem Abschwächer 6 sowie ein Koaxialkabel 7 und eine Magnetspule 9 als Befeldungsspule verwendet. Zudem sind eine Bedieneinheit 20 und eine Anzeigeeinheit 21 mit einem Mikrocontroller 18 der die Regeleinheit und die Indikatoreinheit 29 enthält verbunden. Ein Feldstärke-Sollwert wird über den Signalweg 19 hier unmittelbar dem Mikrocontroller 18 zugeführt, von dem über eine Stellleitung 25 ein Stellsignal unmittelbar an den Abschwächer 6 geführt ist. Über einen Richtkoppler 26 und die Indikatoreinheit 29 wird ermittelt, wie die von der Sender-Abschwächereinheit 4 an die Magnetspule 9 gelieferte Leistung abgenommen wird, zur Unterscheidung, ob dämpfendes Gewebe vorhanden ist, oder ob ein Leerlauffall vorliegt, wozu das sog. Stehwellenverhältnis (SWR) festgestellt wird, indem Signale über die Leitungen 27, 28 dem Mikrocontroller 18 zugeführt werden. Wird ein Leerlauffall ermittelt, wird auch hier anstelle der sonstigen normalen Regelung der Senderpegel reduziert und damit zur Reduzierung der Feldstärke an der Magnetspule 9 der Abschwächer 6 entsprechend angesteuert, so dass keine unzulässig hohen HF-Störpegel im Umfeld der Magnetspule 9 und damit der Befeldungskapsel 8 im kritischen Leerlauffall auftreten.

## Patentansprüche

1. Befetdungsvorrichtung (1) für die Befeldung von Körperteilen von Lebewesen zu Heilzwecken,
mit einem Befeldungsgerät mit einem Hochfrequenzsender (5) zur Abgabe eines Senderpegels,
mit einer daran angeschlossenen Befeldungskapsel (8), die zumindest eine Magnetspule (9) zur Erzeugung einer dem aktuellen Senderpegel zugeordneten Befeldungs-Feldstärke enthält, wobei die Befeldungskapsel (8) in einer Funktionsposition ausreichend nah am Körpergewebe eines Lebewesens zu dessen Befeldung anliegt oder in einer nicht ausreichend nah am Körpergewebe eines Lebewesens liegenden Leerlaufposition weitgehend unbedämpft in die Umgebung abstrahlt, und
mit einer Kontrolleinheit (12) mit einer Steuer- oder Regeleinheit zur betriebsmäßigen Steuerung oder Regelung der Befeldungs-Feldstärke über den Senderpegel,
mit einer Indikatoreinheit (29), welche die vom Hochfrequenzsender an die Befeldungskapsel (8) abgegebene Sendeleistung mit der Größe der **dadurch** in der Befeldungskapsel (8) erzeugten zugeordneten Befeldungs-Feldstärke direkt oder indirekt vergleicht und ins Verhältnis setzt, wobei sich dieses Verhältnis in der Funktionsposition oder in der Leerlaufposition der Befeldungskapsel unterscheidet und ausgewertet wird, dergestalt, dass bei ermittelter Leerlaufposition die Indikatoreinheit ein Schaltsignal abgibt, mit dem auf einen Sicherheitsbetrieb geschaltet wird, indem die Befeldungsvorrichtung bis zu einer Wiedereinschaltung abgeschaltet oder der Senderpegel und damit die Feldstärke über eine Feldstärke-Begrenzungseinrichtung soweit auf einen festgelegten Wert heruntergefahren wird, dass auch in der Leertaufposition kein unzulässig hoher, von der Befeldungskapsel in die Umgebung abgestrahlter Hochfrequenz-Störpegel (HF-Störpegel) auftritt,
**dadurch gekennzeichnet,**
**dass** die Feldstärke-Begrenzungseinrichtung eine Initialisierungseinheit aufweist, über die in einem Initialisierungsmodus nach dem Einschalten des Befeldungsgeräts der HF-Sender (5) gesteuert mit einem so geringen vorgegebenen Initialsenderpegel sendet, dass in keinem der beiden Betriebszustände, nämlich in der Funktionsposition oder Leerlaufposition der Befeldungskapsel, ein unzulässig hoher HF-Störpegel beim Einschalten an der Befeldungskapsel überschritten wird, wobei dem Initialsenderpegel in der Funktionsposition der Befeldungskapsel eine bestimmte niedrig bedämpfte Initial-Feldstärke und in der Leerlaufposition eine bestimmte höhere Leerlauf-Initial-Feldstärke zugeordnet sind,
**dass** eine Grenzwerteinheit vorgesehen ist, der ein Feldstärke-Istwert zugeführt ist und an der ein Grenzwert zwischen der bedämpften Initial-Feldstärke und der Leerlauf-Initial-Feldstärke eingestellt ist,
**dass** bei einer Überschreitung des Grenzwerts im Initialisierungsmodus eine optische und/oder akustische Warnanzeige erfolgt, die einen Benutzer auf die nicht ausreichend nah am Körpergewebe anliegende Befeldungskapsel hinweist, und
**dass** bei einer Unterschreitung des Grenzwerts im Initialisierungsmodus entsprechend einer am Körpergewebe ausreichend nah anliegenden Befeldungskapsel der Initialisierungsmodus beendet wird und eine Freigabe der Reglereinheit erfolgt.

2. Befeldungsvorrichtung nach Anspruch 1, **dadurch gekennzeichnet, dass** beim Einschalten des Befeldungsgeräts eine Zeitschaltung aktivierbar ist, die nach einer eingestellten Befeldungszeit das Befeldungsgerät wieder selbsttätig abschaltet und mit der ein Initialisierungsmodus aktivierbar ist.

3. Befeldungsvorrichtung nach Anspruch 1 oder 2, **dadurch gekennzeichnet, dass** in der Befeldungskapsel zugeordnet ein Feldstärkesensor (10; 26) zur Ermittlung des Feldstärke-Istwerts der Magnetspule (9) vorgesehen ist, welcher an die Indikatoreinheit (29) und gegebenenfalls als Feldstärke-Istwertgeber an eine Regeleinheit angeschlossen ist, und dass ein Pegelsensor (24) zur Ermittlung des aktuellen Senderpegel-Istwerts vorgesehen ist, der an die Indikatoreinheit (29) für einen Vergleich mit dem zugeordneten Feldstärke-Istwert angeschlossen ist, wobei aus dem Vergleichsergebnis die Funktionsposition oder Leerlaufposition der Befeldungskapsel (8) ermittelt wird.

4. Befeldungsvorrichtung nach Anspruch 3, **dadurch gekennzeichnet, dass** der Feldstärkesensor (10) in der Befeldungskapsel (8) angeordnet ist und damit der elektrische Strom und/oder die elektrische Spannung der Magnetspule (9) ermittelt werden, wobei die Werte linear oder logarithmisch gleichgerichtet auf einem Signalweg (11) zur Kontrolleinheit (12) an die Indikatoreinheit (29) geleitet werden.

5. Befeldungsvorrichtung nach einem der Ansprüche 1 bis 4, **dadurch gekennzeichnet, dass** in Verbindung mit der Indikatoreinheit (29) ein Feldstärkesensor und ein Pegelsensor zu einer mittelbaren und indirekten Ermittlung der Feldstärke im Vergleich zum Senderpegel durch einen am HF-Ausgang des Befeldungsgeräts angeordneten Richtkoppler (26) als Stehwellenmessgerät zur Erfassung eines sog. Stehwellenverhältnisses (SWR) gebildet ist, wobei anhand des Stehwellenverhältnisses feststellbar ist, ob die Befeldungskapsel (8) in der Funktionsposition die HF-Sendeleistung gewebegedämpft oder in der Leerlaufposition nicht gewebegedämpft abnimmt und wobei nach Feststellung eines Leerlauffalls eine Ansteuerung eines Abschwächers (6) zur Reduzierung des Senderpegels erfolgt oder die Befeldung selbsttätig abgeschaltet wird.

6. Befeldungsvorrichtung nach Anspruch 5, **dadurch gekennzeichnet, dass** die Kontrolleinheit (12, 18) eine Reglereinheit enthält, der der Feldstärke-Istwert und ein mit einem Sollwertsteller vorgebbarer Feldstärke-Sollwert zugeführt werden, und die durch einen Soll-Istwert-Vergleich über einen vorgebbaren Regelalgorithmus ein Stellsignal abgibt, und
dass eine Sender-Abschwächer-Einheit (4) mit dem Abschwächer (6) als Stellglied vorgesehen ist, dem das Stellsignal bei einer von der Indikatoreinheit freigebenden Regelung zugeführt wird dergestalt, dass bei einer Soll-Istwert-Abweichung mit gegenüber dem Sollwert höherem Istwert der aktuelle Senderpegel und damit die aktuelle Istwert-Feldstärke reduziert werden und umgekehrt bei vergleichsweise kleinerem Istwert der aktuelle Senderpegel vergrößert wird.

7. Befeldungsvorrichtung nach einem der Ansprüche 1 bis 6, **dadurch** ge- /6 kennzeichnet, dass zwei oder mehrere Befeldungskapseln (8) mit jeweils zugeordneten Kontrolleinheiten (12; 18) mit Indikatoreinheiten (29) verwendet sind.

8. Befeldungsvorrichtung nach einem der Ansprüche 1 bis 7, **dadurch gekennzeichnet, dass** der HF-Oszillator (2) ein HF-Signal mit einer vorgebbaren Frequenz im Bereich von 100 MHz bis 200 MHz, bevorzugt von ca. 150 MHz erzeugt, und
dass bevorzugt das HF-Signal mit einer vorgebbaren Niederfrequenz moduliert und/oder mit einer vorgebbaren Taktfrequenz getaktet ist.

## Claims

1. Field-emitting device (1) for irradiating body parts of living beings for therapeutic purposes,
with a field-emitting instrument with a radiofrequency transmitter (5) for emitting a transmitter level,
with a field-emitting capsule (8) connected thereto, which field-emitting capsule contains at least one magnetic coil (9) for generating a radiation-field field strength that is associated with the current transmitter level, with the field-emitting capsule (8), in a functional position, being situated sufficiently closely adjacent to the body tissue of a living being in order to irradiate the latter or said field-emitting capsule radiating largely undamped into the surroundings in an idle position that is not situated sufficiently closely to the body tissue of a living being, and
with a control unit (12) with an open- or closed-loop control unit for operational open- or closed-loop control of the radiation-field field strength via the transmitter level,
with an indicator unit (29), which directly or indirectly compares the transmission power,
emitted from the radiofrequency transmitter to the field-emitting capsule (8), to the magnitude of the associated radiation-field field strength generated thereby in the field-emitting capsule (8) and sets these in relation to one another, wherein this relation differs and is evaluated in the functional position or in the idle position of the field-emitting capsule such that if the idle position is established, the indicator unit emits a switching signal used to switch into safety operation by switching off the field-emitting device until it is switched on again or by reducing the transmitter level, and hence the field strength, using a field-strength limiting apparatus to a predetermined value to the extent that there is no inadmissibly high radiofrequency noise level (RF noise level) that the field-emitting capsule radiates into the surroundings, even in the idle position,
**characterized**
**in that** the field-strength limiting apparatus has an initialization unit, by means of which the RF transmitter (5) transmits in a controlled fashion at such a low predetermined initial transmitter level in an initialization mode after switching on the field-emitting instrument that an inadmissibly high RF noise level is exceeded at the field-emitting capsule during switch-on in neither of the two operational states of the field-emitting capsule, i.e. the functional position or the idle position, wherein a specific slightly damped initial field strength is assigned to the initial transmitter level in the functional position of the field-emitting capsule and a specific higher idle initial field strength is assigned thereto in the idle position,
**in that** provision is made for a limit value unit, which is fed with a field-strength actual value and used to set a limit value between the damped initial field strength and the idle initial field strength,
**in that** there is an optical and/or acoustic alarm indication if the limit value is exceeded in the initialization mode, which indicates to a user that the field-emitting capsule is not sufficiently closely adjacent to the body tissue, and
**in that** if the limit value is undershot in the initialization mode corresponding to a field-emitting capsule being sufficiently closely adjacent to the body tissue then the initialization mode is ended and the closed-loop control unit is enabled.

2. Field-emitting device according to Claim 1, **characterized in that** a timer can be activated when the field-emitting instrument is switched on, which timer automatically switches the field-emitting instrument off again after a set irradiation time and can activate an initialization mode.

3. Field-emitting device according to Claim 1 or 2, **characterized in that**, assigned in the field-emitting capsule, provision is made for a field-strength sensor (10; 26) for establishing the field-strength actual value from the magnetic coil (9), which sensor is connected to the indicator unit (29) and, optionally, to a closed-loop control unit as field-strength actual value transmitter, and
**in that** provision is made for a level sensor (24) for establishing the current transmitter-level actual value, which level sensor is connected to the indicator unit (29) for a comparison with the assigned field-strength actual value, with the functional position or idle position of the field-emitting capsule (8) being established from the result of the comparaison.

4. Field-emitting device according to Claim 3, **characterized in that** the field-strength sensor (10) is arranged in the field-emitting capsule (8) and it is used to establish the electric current and/or the electric voltage of the magnetic coil (9), wherein the values, linearly or logarithmically rectified on a signal path (11) to the control unit (12), are routed to the indicator unit (29).

5. Field-emitting device according to one of Claims 1 to 4, **characterized in that**, in conjunction with the indicator unit (29), a field-strength sensor and a level sensor are formed for indirectly establishing the field strength compared to the transmitter level by means of a directional coupler (26), arranged at the RF output of the field-emitting instrument, as a so-called standing-wave measurement instrument for recording a so-called standing wave ratio (SWR), wherein the standing wave ratio makes it possible to determine whether the field-emitting capsule (8) taps the RF transmission power damped by tissue in the functional position or undamped by tissue in the idle position, and wherein a damper (6) is actuated if an idle case is detected in order to reduce the transmitter level or the irradiation is automatically switched off.

6. Field-emitting device according to Claim 5, **characterized in that** the control unit (12, 18) contains a closed-loop control unit, which is fed with the field-strength actual value and a field-strength setpoint value that can be determined by a setpoint generator, and which emits an actuating signal via a predeterminable closed-loop control algorithm by means of a setpoint/actual-value comparison, and
**in that** a transmitter-damper unit (4) with the damper (6) is provided as an actuator, to which the actuating signal is fed in the case of closed-loop control enabled by the indicator unit such that the current transmitter level and hence the current actual-value field strength are reduced in the case of a setpoint/actual-value deviation with an actual value that is greater than the setpoint value and, vice versa, the current transmitter level is increased if the actual value is comparatively smaller.

7. Field-emitting device according to one of Claims 1 to 6, **characterized in that** use is made of two or more field-emitting capsules (8) with respectively assigned control units (12; 18) with indicator units (29).

8. Field-emitting device according to one of Claims 1 to 7, **characterized in that** the RF oscillator (2) generates an RF signal with a predeterminable frequency in the range between 100 MHz and 200 MHz, preferably approximately 150 MHz, and
**in that**, preferably, the RF signal is modulated with a predeterminable low frequency and/or clocked with a predeterminable clock frequency.

## Revendications

1. Dispositif d'irradiation (1) pour irradier des parties du corps d'êtres vivants à des fins thérapeutiques, comprenant
un appareil d'irradiation avec un émetteur haute fréquence (5) pour délivrer un niveau d'émission,
une capsule d'irradiation (8) connectée à celui-ci et contenant au moins une bobine magnétique (9) pour générer une intensité de champ d'irradiation attribuée au niveau d'émission actuel, dans laquelle la capsule d'irradiation (8), dans une position de fonctionnement, se trouve suffisamment près du tissu corporel d'un être vivant pour irradier celui-ci, ou bien, dans une position en circuit ouvert qui ne se trouve pas suffisamment près du tissu corporel d'un être vivant, rayonne de façon largement non amortie dans l'environnement, et
une unité de contrôle (12) avec une unité de commande ou de régulation pour une commande ou régulation opérationnelle de l'intensité de champ d'irradiation par l'intermédiaire du niveau d'émission,
une unité d'indication (29) qui compare directement ou indirectement la puissance d'émission délivrée par l'émetteur haute fréquence à la capsule d'irradiation (8) à la grandeur de l'intensité de champ d'irradiation attribuée ainsi générée dans la capsule d'irradiation (8) et établit un rapport entre celles-ci, ce rapport étant différent dans la position de fonctionnement ou dans la position en circuit ouvert de la capsule d'irradiation et évalué de telle sorte que lorsque la position en circuit ouvert est déterminée, l'unité d'indication délivre un signal de commutation qui permet de commuter sur un mode de sécurité dans lequel le dispositif d'irradiation est mis hors service jusqu'à ce qu'il soit remis sous tension, ou bien le niveau d'émission, et donc l'intensité de champ, est réduit(e) par un équipement de limitation d'intensité de champ à une valeur fixée à tel point que même dans la position en circuit ouvert, aucun niveau parasite haute fréquence (niveau parasite HF) inadmissible, émis par la capsule d'irradiation vers l'environnement, n'apparaisse,
**caractérisé en ce que**
l'équipement de limitation d'intensité de champ présente une unité d'initialisation par laquelle, dans un mode d'initialisation, après la mise sous tension de l'appareil d'irradiation, l'émetteur HF (5) est commandé à un niveau d'émission initial prédéfini tellement faible que dans aucun des deux modes de fonctionnement, notamment dans la position de fonctionnement ou la position en circuit ouvert de la capsule d'irradiation, un niveau parasite HF inadmissible n'est dépassé lorsque la capsule d'irradiation est mise sous tension, dans lequel le niveau d'émission initial se voit attribuer dans la position de fonctionnement de la capsule d'irradiation une certaine intensité de champ initiale faiblement amortie et dans la position en circuit ouvert une certaine intensité de champ initiale en circuit ouvert supérieure,
**en ce qu'**une unité de valeur limite est prévue à laquelle est amenée une valeur réelle d'intensité de champ et au niveau de laquelle est réglée une valeur limite entre l'intensité de champ initiale amortie et l'intensité de champ initiale en circuit ouvert,
**en ce qu'**en cas de dépassement de la valeur limite en mode initialisation, un affichage d'avertissement optique et/ou acoustique a lieu qui signale à un utilisateur que la capsule d'irradiation ne se trouve pas suffisamment près du tissu corporel, et
**en ce qu'**en cas de soupassement de la valeur limite en mode initialisation, selon une capsule d'irradiation se trouvant suffisamment près du tissu corporel, le mode d'initialisation est terminé et une libération de l'unité de régulation a lieu.

2. Dispositif d'irradiation selon la revendication 1, **caractérisé en ce qu'**à la mise sous tension de l'appareil d'irradiation, un circuit de synchronisation peut être activé qui remet l'appareil d'irradiation automatiquement hors tension à l'issue d'un délai d'irradiation réglé et qui permet d'activer un mode d'initialisation.

3. Dispositif d'irradiation selon la revendication 1 ou 2, **caractérisé en ce que** dans la capsule d'irradiation, un capteur d'intensité de champ (10 ; 26) est prévu par attribution pour déterminer la valeur réelle d'intensité de champ de la bobine magnétique (9) qui est connectée à l'unité d'indication (29) et le cas échéant à une unité de régulation comme générateur de valeur réelle, et
**en ce qu'**un capteur de niveau (24) pour déterminer la valeur réelle de niveau d'émission actuelle est prévu qui est connecté à l'unité d'indication (29) pour une comparaison avec la valeur réelle d'intensité de champ attribuée, la position de fonctionnement ou la position en circuit ouvert de la capsule d'irradiation (8) étant déterminée à partir du résultat de la comparaison.

4. Dispositif d'irradiation selon la revendication 3, **caractérisé en ce que** le capteur d'intensité de champ (10) est disposé dans la capsule d'irradiation (8) et permet de déterminer le courant électrique et/ou la tension électrique de la bobine magnétique (9), dans lequel les valeurs discriminées de façon linéaire ou logarithmique sont amenées à l'unité d'indication (29) sur un chemin de signal (11) allant jusqu'à l'unité de contrôle (12).

5. Dispositif d'irradiation selon l'une quelconque des revendications 1 à 4, **caractérisé en ce qu'**en relation avec l'unité d'indication (29), un capteur d'intensité de champ et un capteur de niveau pour une détermination directe et indirecte de l'intensité de champ en comparaison avec le niveau d'émission sont formés par un coupleur directif (26), disposé à la sortie HF- de l'appareil d'irradiation, comme appareil de mesure d'ondes stationnaires en vue de la détection d'un taux dit d'ondulation (SWR), ledit taux d'ondulation permettant de déterminer si la capsule d'irradiation (8) dans la position de fonctionnement prélève la puissance d'émission HF de façon amortie par le tissu ou la prélève de façon non amortie par le tissu dans la position en circuit ouvert, et dans lequel, après détermination d'une situation de circuit ouvert, un réducteur (6) est piloté pour réduire le niveau d'émission ou l'irradiation est automatiquement mise hors tension.

6. Dispositif d'irradiation selon la revendication 5, **caractérisé en ce que** l'unité de contrôle (12, 18) comprend une unité de régulation à laquelle on amène la valeur réelle d'intensité de champ et une valeur de consigne d'intensité de champ prédéfinissable à l'aide du régleur de valeur de consigne et qui délivre par l'intermédiaire d'une comparaison des valeurs de consigne et réelle un signal de réglage par un algorithme de régulation prédéfinissable, et
**en ce qu'**une unité émetteur/réducteur (4) avec le réducteur (6) est prévue comme un actionneur auquel on amène le signal de réglage en cas de régulation validée par l'unité d'indication, de telle sorte qu'en cas d'écart entre les valeurs de consigne et réelle avec une valeur réelle supérieure à la valeur de consigne, le niveau d'émission actuel et donc l'intensité de champ réelle actuelle sont réduits, et inversement, avec une valeur réelle relativement inférieure, le niveau d'émission actuel est augmenté.

7. Dispositif d'irradiation selon l'une quelconque des revendications 1 à 6, **caractérisé en ce que** deux ou plusieurs capsules d'irradiation (8) avec des unités de contrôle (12 ; 18) respectivement attribuées avec des unités d'indication (29) sont utilisées.

8. Dispositif selon l'une quelconque des revendications 1 à 7, **caractérisé en ce que** l'oscillateur HF (2) génère un signal HF à une fréquence prédéfinissable dans la plage de 100 MHz à 200 MHz, de préférence d'environ 150 MHz, et
**en ce que** de préférence le signal HF est modulé sur une basse fréquence prédéfinissable et/ou synchronisé sur une fréquence d'horloge prédéfinissable.
